Europäisches Patentamt

European Patent Office

Office européen des brevets

(19)

(11) **EP 1 159 448 B1**

(12) **EUROPEAN PATENT SPECIFICATION**

(45) Date of publication and mention
of the grant of the patent:
**14.04.2004 Bulletin 2004/16**

(51) Int Cl.⁷: $C12Q\ 1/56$, G01N 33/86, C12Q 1/37

(21) Application number: **00914107.8**

(22) Date of filing: **03.03.2000**

(86) International application number:
**PCT/EP2000/001912**

(87) International publication number:
**WO 2000/052199 (08.09.2000 Gazette 2000/36)**

(54) **DETERMINATION OF BIOLOGICALLY ACTIVE FORMS OF PROTEOLYTIC ENZYMES**

BESTIMMUNG VON BIOLOGISCH AKTIVEN FORMEN VON PROTEOLYTISCHEN ENZYMEN

DETERMINATION DES FORMES BIOLOGIQUEMENT ACTIVES D'ENZYMES PROTEOLYTIQUES

(84) Designated Contracting States:
**AT BE CH CY DE DK ES FI FR GB GR IE IT LI LU MC NL PT SE**

(30) Priority: **04.03.1999 EP 99200627**
**01.10.1999 US 157240 P**

(43) Date of publication of application:
**05.12.2001 Bulletin 2001/49**

(73) Proprietor: **Synapse B.V.**
**6200 MD Maastricht (NL)**

(72) Inventors:
• **HEMKER, Hendrik, Coenraad**
**NL-6211 LM Maastricht (NL)**
• **WAGENVOORD, Robert, Jan**
**NL-6229 KW Maastricht (NL)**
• **RAMJEE, Manoj**
**Cambridge CB4 4WG (GB)**

(74) Representative: **Huygens, Arthur Victor et al**
**Octrooibureau Huygens,**
**P.O. Box 86**
**3400 AB Ijsselstein (NL)**

(56) References cited:
WO-A-96/21740          US-A- 3 985 620
US-A- 4 897 444         US-A- 5 073 487

• HEMKER HC, WILLEMS GM, BEGUIN S: "A computer assisted method to obtain the prothrombin activation velocity in whole plasma independent of thrombin decay processes" THROMBOSIS AND HAEMOSTASIS, vol. 56, no. 1, 20 August 1986 (1986-08-20), pages 9-17, XP000567420 cited in the application
• HEMKER HC, WIELDERS S, KESSELS H, BEGUIN S: "Continuous registration of thrombin generation in plasma, its use for the determination of the thrombin potential" THROMBOSIS AND HAEMOSTASIS, vol. 70, no. 4, 18 October 1993 (1993-10-18), pages 617-624, XP000567560 cited in the application
• TRAVIS J, SALVESEN GS: "Human plasma proteinase inhibitors" ANNUAL REVIEW OF BIOCHEMISTRY, vol. 52, 1983, pages 655-709, XP002110610 cited in the application
• BARRETT AJ: "Alpha 2-macroglobulin" METHODS IN ENZYMOLOGY, vol. 80, 1981, pages 737-754, XP002110611 cited in the application

## Description

## Field of the Invention

The present invention is in the field of diagnostics and relates more in particular to a method for the determination of biologically active forms of proteolytic enzymes, such as thrombin, in blood and other fluids.

## Background of the Invention

In the Western world, arterial and venous thrombosis and atherosclerosis together are currently responsible for well over 50% of all mortality and serious morbidity. In a few decades this will be the case worldwide [Murray, C.J. and A.D. Lopez, Science (1996) **274**: 740-743]. Thrombosis is caused by an overactivity of the haemostatic mechanism that is responsible for the arrest of bleeding from a wound. This haemostatic-thrombotic system (HTS) is a complex interaction between vessel wall, blood cells, especially blood platelets, and plasma proteins. See, e.g., Hemker H.C. *Thrombin generation, an essential step in haemostasis and thrombosis.* In: Haemostasis and thrombosis, A.L. Bloom *et al.*, eds., Churchill Livingstone, Edinburgh, (1994) 477-490.

In blood there are at least three series of proenzyme-enzyme cascades of great biological importance: the blood coagulation system, the fibrinolytic system and the complement system. In each of these systems proteolytic proenzymes are activated and subsequently inhibited. In general it is important that the concentration of an activated enzyme in such a cascade can be measured in order to assess the function of the system. In the following a typical description of the clotting system will be given with an emphasis on its most important enzyme, thrombin. It is to be noted, however, that the present invention also rolates to other clotting enzymes and enzymes of the fibrinolytic and complement pathway.

The mechanism of thrombin generation can be outlined in some detail as follows. In the plasmatic coagulation system factor Xa is formed by the action of the tissue factor-factor VIIa complex (TF-VIIa). Factor Xa binds to tissue factor pathway inhibitor (TFPI) and the TFPI-Xa complex inhibits the TF-VIIa complex. Thrombin activates factors V, VIII and XI and so accelerates its own generation, but it also binds to thrombomodulin and so starts the protein C mechanism that breaks down activated factors V and VIII, thus, indirectly, inhibiting further thrombin generation. An important fraction ($\approx$30%) of all thrombin formed in clotting plasma is bound to the fibrin clot. Clot-bound thrombin does retain its thrombotic properties, it can clot fibrinogen, activate factors V, VIII and XI as well as platelets [Béguin, S. and R. Kumar, Thromb. Haemost. (1997) **78**: 590-594; Kumar, R., S. Béguin, and H.C. Hernker, Thromb. Haemost. (1994) **72**:713-721, and (1995) **74**: 962-968]. It is not inhibited by antithrombin.

Thrombin action causes receptors in the platelet membrane to bind fibrinogen, which causes platelet aggregation. Platelet activation also leads to the exposure of procoagulant phospholipids in a Von Willebrand factor dependent reaction [Béguin S., R. Kumar, I. Keularts, U. Seligsohn, B.C. Coller and H.C. Hemker, *Fibrin-Dependent Platelet Procoagulant Activity Requires GPIb Receptors and Von Willebrand Factor*, Blood (1999) 93:564-570; Béguin, S. and R. Kumar, *supra* (1997)]. These phospholipids are required for the proper activation of factor X and prothrombin. Recently, the picture has been complicated by the discovery that fibrin, previously thought to be the inert endproduct of coagulation, plays an active role itself. It binds and activates Von Willebrand factor, which activates platelets and provokes the exposure of procoagulant phospholipids via an alternative pathway.

The cooperation between platelets and the coagulation system, including fibrin, is central to the haemostatic-thrombotic system. The mechanism shows an abundance of positive and negative, often nested, feedback loops. Underactivity causes bleeding, overactivity causes thrombosis. Thrombosis manifests itself as coronary infarction, stroke, pulmonary embolism and a large number of less frequent diseases.

In order to assess the function of such a system, also for diagnostic purposes and for the safe use of antithrombotic drugs, a probe is needed for the functional status of the haemostatic-thrombotic system. An important function test of the HTS is the thrombin generation curve (TGC). Carried out in platelet-poor plasma, it gives information about the function of the plasmatic clotting system. In platelet-rich plasma it measures also the function of the platelets.

According to the prior art the TGC can be measured by subsampling or continuously. In the ancient subsampling method [Biggs, R. and R.G. Macfarlane, *Human Blood Coagulation and its Disorders.* 1953, Oxford: Blackwell; Quick, A.J., *Haemorrhagic Diseases.* 1957, Philadelphia: Lea & Febiger], samples are taken from a clotting mixture, and the concentration of thrombin is measured in each sample.

Active thrombin survives in plasma for only a limited period of time (the half life time is 16-17 s). This is due to circulating antithrombins. Most thrombin (64%) is inactivated by antithrombin (AT), a plasma-protein of 57 kD, 23% by $\alpha_2$-macroglobulin ($\alpha_2$M), a 725 kD plasma-protein, and 13% by various other agents [Hemker, H.C., G.M. Willems, and S. Béguin, *A computer assisted method to obtain the prothrombin activation velocity in whole plasma independent of thrombin decay processes,* Thromb. Haemost. (1986) **56**:9-17]. The $\alpha_2$-macrogiobulin-thrombin complex ($\alpha_2$M-IIa) has the peculiarity that it is inactive towards all macromolecular substrates, but retains its activity against small molecular weight (artificial) substrates. $\alpha_2$-Macroglobulin is a glycoprotein of Mr 725,000, which is present in plasma in a concentration of 2500 mg/L or 3.5 $\mu$M. It is a tetramer of identical subunits of 185 kD. The inactivation reaction of an active proteinase or activated clotting factor with $\alpha_2$M is a three step process: 1°. Formation of a loose complex, 2°. Hydrolysis of a target peptide in $\alpha_2$M, causing 3°. a rapid conformational change which physically entraps the enzyme molecule within the $\alpha_2$M molecule. [See for a review: Travis, J. and G.S. Salvesen, *Human plasma proteinase inhibitors*. Ann. Rev. Biochem. (1983) **52**: 655-709].

The group of Hemker then developed a continuous method in which thrombin-catalysed product formation from suitable substrates is monitored directly [Hemker, H.C., *et al., Continuous regisfration of thrombin generation in plasma, its use for the detennination of the thrombin potential*. Thromb. Haemost. (1993) **70**:617-624]. The kinetic constants of the substrate are such that, at the concentration used, the rate of product formation is proportional to the amount of enzyme present (thrombin or $\alpha_2$M-IIa). The time-course of enzyme activity in the sample can be estimated as the first derivative of the product-time curve.

A drawback of this method is that part of the thrombin in plasma binds to $\alpha_2$-macroglobulin. $\alpha_2$-Macroglobulin is the most abundant non-specific protease inhibitor of blood plasma. It quenches the biological activity of proteases (activated clotting factors, activated fibrinolytic enzymes and activated complement factors) without occupying their active centre, so that there is a residual activity on the usual oligopeptide signal-substrates.

This $\alpha_2$M-IIa complex has no known physiological activity but is able to cleave chromogenic substrates. Thus, the product formation observed is the result of the combined activities of free thrombin and $\alpha_2$-macroglobulin-bound thrombin. The relevant data, i.e. the amount of product formed by free thrombin only, can be extracted from the experimental product formation by a mathematical operation. However, the operation has to be carried out on the whole course of the product generation curve. The product formation therefore needs to be monitored continuously. Although the principle of this continuous method is applicable to all substrates that give a product which can be detected in time, e.g. by fluorescent, electrochemical or NMR signals, its application is currently restricted to the use of chromogenic substrates in an optically clear medium, i.e. defibrinated platelet poor plasma (PPP).

Therefore, there is still a need for an efficient method to measure the amount of (active forms of) proteolytic enzymes, such as thrombin, in blood and other fluids, which obviates the drawbacks of the prior art. The present invention provides such a method.

WO 96/21740 discloses a method for determining the endogenous thrombin potential (ETP) of a sample, preferably in a continuous assay, which comprises contacting a sample to be assessed with a water soluble synthetic substrate that comprises a leaving group generating a detectable signal upon cleavage.

ETP is defined as the surface under thrombin-time curve and is believed by the authors to be the parameter that best renders the thrombin-generating capacity of a plasma sample.

US-A-3985620 discloses certain substrates for determining proteases wherein a substrate protein is bound by a covalent bond in fine distribution in or on the surface of a solid carrier and dyed with a reactive dyestuff. Preferred substrates are haemoglobin, casein fibrinogen, collagen, and modification products of these proteins. The cleavage of the substrate by a complexed protease is not addressed.

## Summary of the Invention

In accordance with the present invention, a process is provided for the assessment of an active proteolytic enzyme in a blood or another biological fluid sample possibly comprising a complex of said proteolytic enzyme and $\alpha_2$-macroglobulin, wherein said sample is contacted with a substrate comprising a molecule having a $M_r$ larger than 10kD coupled to a signal-substrate, said signal-substrate comprising a detectable leaving group, wherein said substrate is hydrolysed by said proteolytic enzyme but not by said complex.

The active proteolytic enzyme is preferably selected from the group consisting of thrombin, activated clotting factor, activated fibrinolytic factor, and activated component of the complement system. Of these, thrombin is most preferred.

The molecules of sufficient size are preferably selected from the group consisting of inert protein, preferably ovalbumin, polysaccharide, and synthetic polymer. Preferably, these inert molecules are water soluble and have a size such that they just do not fit into the cavity of the $\alpha_2$M molecules. A group of preferred inert molecules has a size of about 40 kD and a solubility of at least 40 mg/ml. Most preferred are inert molecules with a size of about 20 kD and a solubility of at least 50 mg/ml.

These and other aspects of the invention will be further outlined in the description which follows.

## Brief Description of the Drawings

Figure 1 represents theoretical curves of hydrolysis products formed in clotting plasma or blood by hydrolysis of a small thrombin substrate and a large thrombin substrate.
Figure 2 represents the curves of hydrolysis products of substrate S2288 coupled with ovalbumin by hydrolysis with $\alpha_2$M-IIa and thrombin, respectively.
Figure 3 represents the fluorescence patterns of the fluorescent substrate Ala-Arg-AMC coupled with ovalbumin by hydrolysis with free thrombin and thrombin inactivated by $\alpha_2$M, respectively.

Figure 4 shows the results of the determination of the ETP in plasma with SQ68 and OA-Phe-Pip-Lys-pNA.

## Detailed Description of the Invention

The present invention is based on the surprising finding that certain signal-substrates for proteolytic enzymes, notably thrombin substrates, are cleft by thrombin but not by the $\alpha_2$M-IIa complex. Thus, these signal-substrates are insensitive to the action of proteolytic enzymes when they are bound to $\alpha_2$-macroglobulin.

As used herein, the term "signal-substrate" means a substrate that is cleaved by proteolytic enzyme (s) present in the medium, from which a leaving group is split off which is detectable by optical, NMR or other methods. Leaving groups which are optical detectable are, for example, p-nitroanilide and 7-amido-4-methylcoumarin. p-Nitroanilide absorbs at 405 nm and 7-amido-4-methylcoumarin is fluorescent (excitation at 390 nm and emission at 460 nm). Examples of NMR-active leaving groups are those containing $^{31}$P, $^{13}$C, or any other atom which can be detected with NMR or a similar technique. Also H$^+$ ions can be used as leaving group, which can be detected by measuring changes in the pH.

The present invention has the advantage that the total transient enzymatic activity of the free thrombin in the clotting sample can be assessed from the total amount of substrate that is converted, so that the mathematical procedure can be omitted.

The formation of thrombin in clotting blood or plasma arises from prothrombin by an enzymatic controlled reaction, which is called prothrombinase. Initially this reaction is slow, but the rate increases exponentially until the formed thrombin activates the inactivators of the prothrombinase and the prothrombin is exhausted.

We have found that the formation of thrombin can be fitted into the following mathematical equation:

$$FIIa_t = PT \cdot (1 - e^{-b*t})c \qquad (i)$$

in which FIIa$_t$ is the formation of thrombin in time, PT is the initial amount of prothrombin, b and c are constants and t is the time. By fitting an actually measured thrombin generation curve with this formula one finds a very good agreement between the fit and the data when b = 0.02 and c = 4.

The formed thrombin will react with the plasmatic inhibitors, from which AT and $\alpha_2$M are the most important. The reaction constants (decay constants) of these reaction are about 20,000 (M.s)$^{-1}$ and 1,500 (M.s)$^{-1}$, respectively.

If the amount of thrombin is to be measured continuously, a thrombin substrate is added to the reaction mixture. By the action of thrombin a molecule is split off from the substrate which can be measured, for example in a spectrophotometer or a fluorometer.

It has now been found, after extensive research and experimentation, that in contrast to small substrates, when using a relatively bulky thrombin substrate, hydrolysis stops when all thrombin has been inactivated, and the total enzymatic activity of thrombin can be measured as an end-point assay.

Accordingly, in one aspect of the present invention there are provided large size chromogenic and fluorogenic and other signal-substrates, to the extent that they cannot penetrate into the cavity of the $\alpha_2$M molecule and thus are not hydrolysed by $\alpha_2$M-IIa. This is achieved by coupling the signal-substrate to an inert bulky carrier molecule which results into a "substrate". The coupling is preferably carried out chemically, in a way which is known to a man skilled in the art.

The exclusion limit of the cavity of $\alpha_2$M in which an active protease is entrapped is about 10 kD (Barrett, A., *Methods in Enzymol.* (1981) **80**:737-754]. Thus, suitable inert carrier molecules are larger than 10 kD and include, for example, proteins, polysaccharides, synthetic polymers. Preferably, the inert molecules have a good solubility. One preferred group of inert molecules has a size such that the molecules just do not fit into the cavity of the $\alpha_2$M molecule. Another group of preferred inert molecules has a size of about 40 kD and a solubility of at least 40 mg/ml. Most preferred are inert molecules with a size of about 20 kD and a solubility of at least 50 mg/ml.

Suitable and preferred proteins are those which are commercially available, such as ovalbumin (Mr = 45 kD), bovine serum albumin (Mr = 67 kD), human serum albumin (Mr = 67 kD) and hen-egg white lysozyme (Mr = 14.4 kD).

Suitable polysaccharides have a Mr which may vary from a few kD (so that they do not fit into the $\alpha_2$M cavity) to more than 500 kD. An example of such a polysaccharide is dextran.

Suitable synthetic polymers include hydrophilic water soluble polymers, with a Mr also varying from a few kD to more than 500 kD, such as polyacrylic acid, polyethylene oxide, polyvinyl chloride, poly(1-vinylpyrrolidone-co-acrylic acid), poly(2-hydroxyethyl methacrylate), and the like.

Suitable signal-substrates for the purpose of the present invention are compounds with a leaving group which can be easily detected and which is not split by the $\alpha_2$-macroglobulin-protease complex. The leaving group preferably gives an optical density signal or a fluorescent signal. Examples of such leaving groups include p-nitroanllide and 7-amino-4-coumarin, respectively.

Suitable substrates for the purpose of the present invention include compounds which are obtained by coupling a signal-substrate of relatively low Mr to a inert molecule of sufficient size. Examples of suitable substrates include ovalbumin coupled to H-D-isoleucyl-L-prolyl-L-arginine-p-nitroanilide.2HCl (also referred to as "OA-S2288"), ovalbumin coupled to H-alanyl-L-arginine-7-amido-4-methyl-coumarin (also referred to as "OA-Ala-Arg-AMC"), and ovalbumin coupled to H-D-phenylalanyl-L-prolyl-L-lysine-p-nitroanilide.2HCl (also referred to as "OA-Phe-Pro-Lys-pNA"). Another example of a suitable substrate is ovalbumin coupled to a signal-substrate releasing one or more hydrogen ions as leaving group.

When such a substrate is used to measure the thrombin generation curve (TGC), the temporary thrombin will hydrolyse a certain amount of substrate, after which no further hydrolysis will take place. The amount of the substrate, as well as the kinetic properties thereof, are preferably so chosen that the amount of thrombin generated in the plasma (or other medium) does not consume the substrate completely. The end-level of conversion product formed can be measured and is directly proportional to the surface under the TGC, which is called the endogenous thrombin potential (ETP).

The substrates can be suitably used to measure the biologically active form of proteolytic enzyrnes in blood and other fluids containing $\alpha_2$-macroglobulin. An important application is the generation and disappearance of thrombin in clotting blood, which is an important function test of the haemostatic-thrombotic system (HTS).

Although the present invention is typically described and exemplified for the assessment of (generated) thrombin in blood and other biological fluids, it will be understood that the method is also suitable for the assessment of other proteolytic enzymes in such biological fluids using possible variations and modifications which are clear to the man skilled in the art. Examples of such other proteolytic enzymes include activated clotting factor, activated fibrinolytic factor, and activated component of the complement system.

The invention is further illustrated by the following Examples which should not be construed as limiting the invention in any respect.

## Example 1

### Hydrolysis of a thrombin substrate in clotting blood or plasma

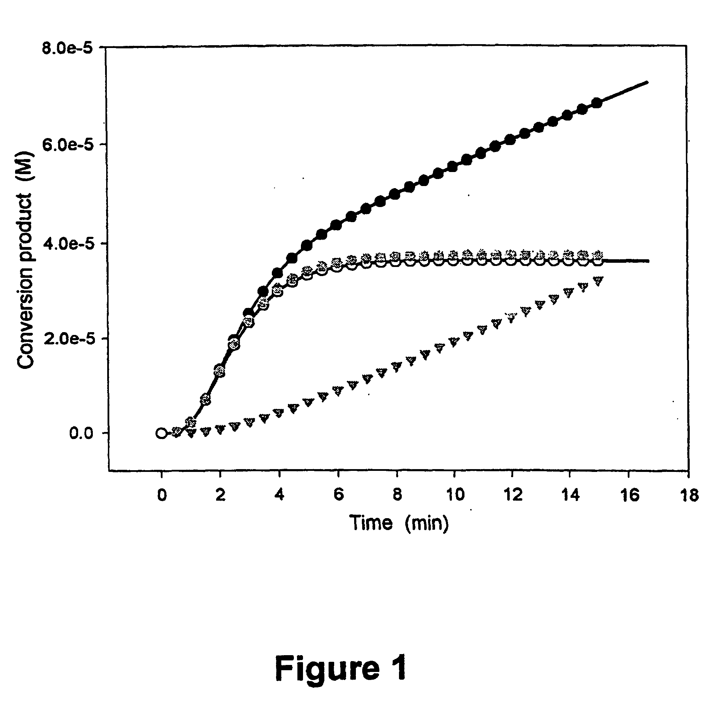

To blood or plasma 0.5 mM thrombin substrate is added and blood coagulation starts by calcification and a suitable trigger, in this example typically thromboplastin. Figure 1 shows the theoretical curves of the formation of conversion products in time.

The curves (-•- or -o-) show the hydrolysis of a thrombin substrate in clotting plasma. We assume that thrombin formation occurs to formula (i), see the Detailed Description, with the constants mentioned therein. The formed thrombin will react with both AT and $\alpha_2$M. The AT concentration is set to 2.5 $\mu$M and the $\alpha_2$M concentration to 3.5 $\mu$M. The reaction constant of thrombin inactivation by AT is 20,000 $(M.s)^{-1}$, and that of thrombin inactivation by $\alpha_2$M is 1,450 $(M.s)^{-1}$. To design the curves numerical methods have been used. We used steps of 1 s. After 1 s an amount of thrombin is formed as indicated by equation (i). From 1 to 2 seconds this amount of thrombin will react with AT and $\alpha_2$M at a rate determined by the above given constants. After this second some AT-thrombin complex (AT-IIa) and $\alpha_2$M-IIa is formed. These amounts are subtracted from the present amount of thrombin, but new thrombin is formed in this second according to equation (i). From 2 to 3 seconds the new amount of thrombin will react with AT and $\alpha_2$M (both corrected for the complexes) while new AT-IIa and $\alpha_2$M-IIa is formed and thus some thrombin is inactivated. This process is repeated during the time span of 15 min. Thus, we see the build up of AT-IIa and $\alpha_2$M-IIa during the process and a temporary increase of thrombin, followed by complete inactivation of thrombin by AT and $\alpha_2$M.

By addition of a small thrombin substrate to this mixture two new reactions are introduced: S is hydrolysed by free thrombin and by $\alpha_2$M-IIa. When S is a large substrate, it will be hydrolysed by the free thrombin, but not by $\alpha_2$M-IIa. These reactions will follow Michaelis-Menten kinetics: hydrolysis rate = [Thrombin] . $k_{cat}$ . S / $(K_m + S)$.

The amount of hydrolysis product is calculated with the numerical procedure described above. In case of curve -•-, S is a small thrombin substrate and in case of curve -o-, S is a large thrombin substrate.

The thrombin substrate (S) is hydrolysed by thrombin with a $K_m$ of 500 $\mu$M and a $k_{cat}$ of 1 $s^{-1}$. Also $\alpha_2$M-IIa hydrolyses S, in case of curve -•- $K_m$ is 500 $\mu$M and $k_{cat}$ is 0.5 $s^{-1}$, and in case of curve -o- $K_m$ is 1 M and $k_{cat}$ is zero (i.e. S is not hydrolysed).

In this Example a model substrate is used with the kinetics given above. In actual practice substrates are available with kinetics which are close to this model. Suitable and preferred examples of such substrates are SQ68, Msc-Val-Arg-pNA, DEMZ-Gly-Arg-pNA, and Petachrome TH/SR.

The above procedure is usually applied in a somewhat modified form in that the process is not monitored continuously but samples are taken every 30 sec and the formed conversion product (indicated by the circles • or o) measured.

When using a small thrombin substrate, which procedure was routinely used prior to the present invention, curves were obtained as shown by -•-. In order to determine the amount of conversion product formed by the action of free thrombin, the curve was split into two parts, product formation by free thrombin and $\alpha_2$M-IIa, respectively. Product formation by free thrombin will stop when all thrombin has been inactivated, but product formation by $\alpha_2$M-IIa will continue. This explains the ongoing linear product formation at the end of curve -•-. The collection of data measured had to be split into one set for product formation by free thrombin and one set for product formation by $\alpha_2$M-IIa. For this procedure a mathematical algorithm has been developed [Hemker, H.C., S. Welders, H. Kessels and S. Béguin, *Continuous registration of thrombin generation in plasma, its use for the determination of the thrombin potential*. Thromb. Haemost. (1973) **70**:617-624]. The principle is the following. At t=0 no thrombin and no $\alpha$2M-IIa is present and the signal is set to zero. At t=0.5 min (second measured data point) an increased signal is measured due to S hydrolysis by thrombin and $\alpha_2$M-IIa. A fraction of this signal (k x $\alpha$2M-IIa concentration) is due to $\alpha_2$M-IIa. At t=1 min, the signal is increased due to S hydrolysis by free thrombin by the present amount of $\alpha_2$M-IIa. This procedure is continued until the last data point is reached. From the theoretically derived curve an the actual observations we know that at the end of the curve all free thrombin has been inactivated and the increase of the signal is completely due to S hydrolysis by $\alpha_2$M-IIa. By choosing the k at which the last part of the curve becomes minimal (i.e. the formation of product is constant in time) the data set can be split into a signal due to S hydrolysis by free thrombin (-o-) and a signal due to the effect of $\alpha_2$M-IIa (-▽-).

It is noted that the signal caused by the free thrombin reaches a plateau which remains the same, independent on the method used. When a small thrombin substrate is applied, this ceiling has to be determined with a mathematical algorithm. However, when a large thrombin substrate is used according to the present invention, the plateau can be easily determined by reading the end-level of signal.

**Example 2**

**Hydrolysis of ovalbumin substrate OA-S2288 by $\alpha_2$M-IIa and thrombin**

In this example it is shown that by coupling of ovalbumin to S2288 the substrate loses its ability to be hydrolysed by $\alpha_2$M-IIa, but not by thrombin. This substrate (OA-S2288) was prepared in the following way. A reaction mixture was prepared containing 1 mM S2288, 50 mM sodium carbonate (pH 10.2), 20 mg/ml ovalbumin and 3.6 mg/ml bis(sulfosuccinimidyl)suberate (BS[3]) This mixture was incubated for 30 min at room temperature, then 50 mM Tris (pH 8.2) was added and the mixture was kept at room temperature for 30 min. The mixture was applied to a Sephadex G25 column (16 x 1.6 cm[3]) and eluted with a rate of 3 ml/min. The elution buffer was 50 mM HEPES, 100 mM NaCl (pH 7.35). The first peak that eluted from the column contained the ovalbumin coupled to the S2288 (OA-S2288).

The amidolytic activity of human thrombin and human $\alpha_2$M-IIa towards OA-S2288 was measured. The reaction mixtures contained 22 µM OA-S2288, 50 nM thrombin or $\alpha_2$M-IIa and 175 mM NaCl, 50 mM Tris-HCl, 2 mg/ml ovalbumin (pH 7.9).

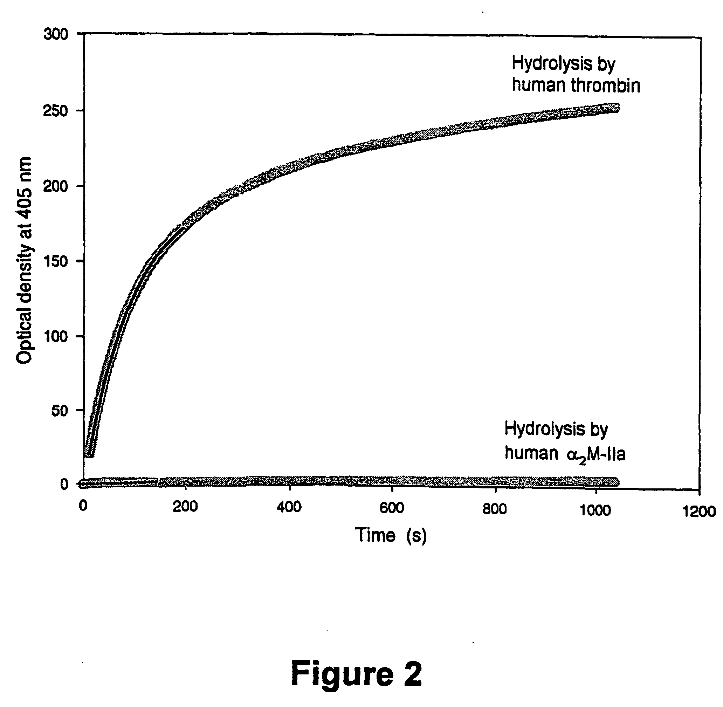

In Figure 2 the amount of free p-nitroanilide (pNA) formed, expressed as milli optical density at 405 nm, is plotted against the time. The reaction temperature was 37°C. The grey curves are the measured data, whereas the black line is obtained by fitting, assuming that product formation occurs according to Michaelis-Menten kinetics.

Product formation in time can be described as: $E \cdot k_{cat} \cdot S_t / (K_m + S_t)$, in which $S_1$ is the substrate concentration in time (i.e. the substrate concentration originally present ($S_0$) minus the formed product or split substrate, and E is the enzyme that splits the substrate, either thrombin or $\alpha_2$M-IIa.

The following constants were found: $S_0 = 22$ µM, $K_m = 1.47$ µM and $k_{cat} = 0.31$ s$^{-1}$ for E is thrombin and 0.002 s$^{-1}$ for E is $\alpha_2$M-IIa.

**Example 3**

**Hydrolysis of ovalbumin substrate OA-Ala-Arg-AMC by free thrombin and thrombin inactivated by $\alpha$2M.**

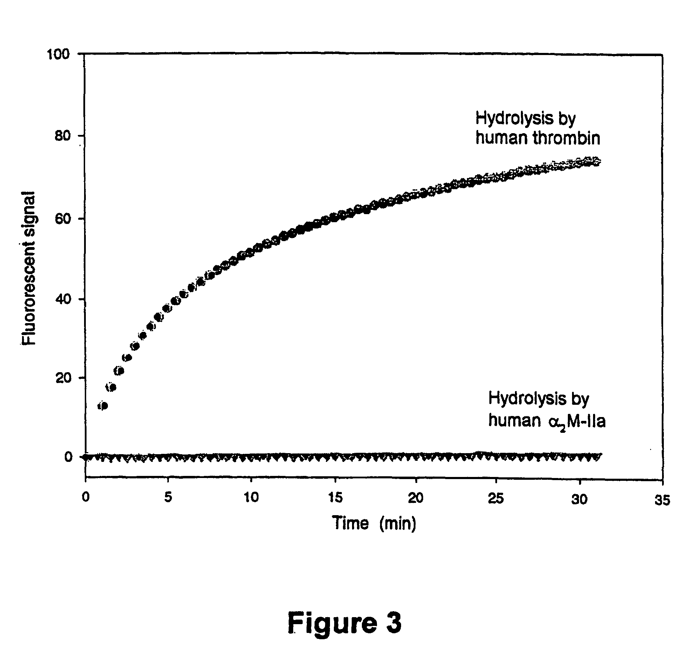

This example shows that by coupling of ovalbumin to the fluorescent substrate Ala-Arg-AMC, the substrate will not be split anymore by thrombin entrapped in the cavity of $\alpha$2M. Figure 3 shows that free thrombin splits off the fluorescent group (AMC), but that thrombin inactivated by $\alpha$2M cannot.

The coupling of ovalbumin to Ala-Arg-AMC was carried out as follows. To 1 ml of a 1 mg/ml ovalbumin in PBS (= 10 mM potassium phosphate, 150 mM NaCl, pH 7.4) was added 6 µl of 50 mM BS[3] in 100% dimethylsulfoxide. The mixture was incubated at room temperature for 60 min. and dialysed twice against 2 L PBS (each for 2h). Then 5 µl of 100 mM Ala-Arg-AMC was added and incubated at room temperature for 60 min. Finally, the sample was dialysed as before (twice against 2 L PBS).

Figure 3 shows the results of fluorescence measurements in a Fluoroskan Acsent equipped with a thermostated block, a 390 nm excitation and 460 nm emission filter. The assay was carried out at 25°C in 96 well Microfluor W "U" bottomed micro titre plates (Dynex, London, UK) in 10 mM HEPES, 5 mM CaCl$_2$, 0.02% sodium azide, pH 8.0). In case of -o- the vessel contained 5.5 µM OA-AR-AMC (Ala-Arg-AMC coupled to ovalbumin) and 85 nM human thrombin and in case of -▽- 5.5 µM OA-AR-AMC and 65 µM human $\alpha$2M-IIa. The black symbols (-•- and -▼-) were obtained by fitting assuming that the substrate is hydrolysed by thrombin according to Michaelis-Menten kinetics. From the fitting the following constants were found for hydrolysis by thrombin: the substrate concentration $S_0 = 5.5$ µM, $K_m = 1.2$ µM and $k_{cat} = 3.3$ s$^{-1}$. For hydrolysis by $\alpha_2$M-IIa the already derived $S_0$ and $K_m$ were found and $k_{cat} = 0.008$ s$^{-1}$.

## Example 4

**Determination of the ETP in plasma with SQ68 and OA-Phe-Pip-Lys-pNA**

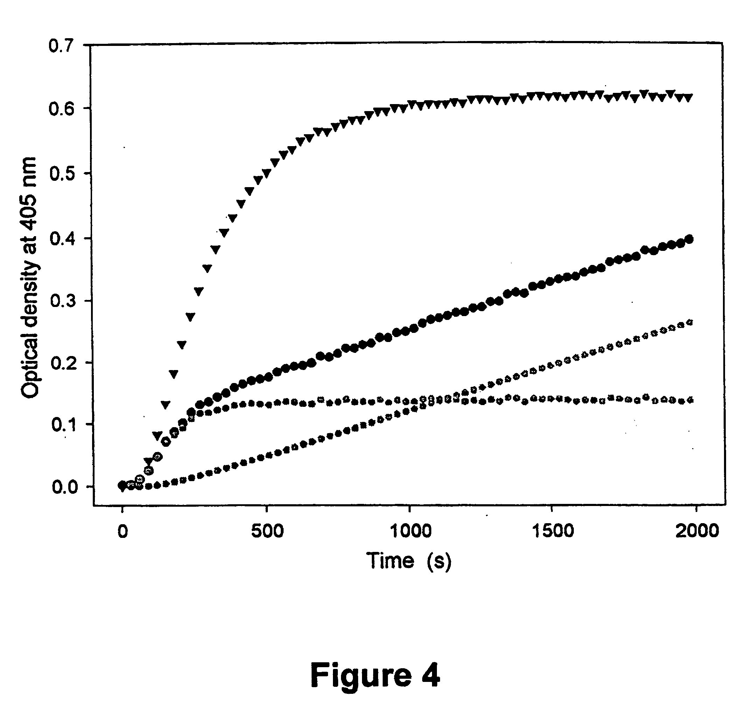

See Figure 4. Thrombin generation was measured as described earlier [Hemker, H.C., Wielders, S., Kessels, H., Béguin, S. *Continuous Registration of Thrombin Generation in Plasma, its Use for the Determination of the Thrombin Potential.* Thromb. Haemost. (1993) **70**:617-624, and Hemker, H.C. and Béguin S. *Thrombin generation in plasma: its assessment via the endogenous thrombin potential.* Thromb. Haemost. (1995) **74**:134-138].

Formation of pNA in clotting plasma to which was added SQ68 (•) or OA-Phe-Pip-Lys-pNA (▼); Pip is pipecolyl. The pNA formation was monitored by measuring the optical density at 405 nm. The substrate concentration was 0.5 mM in both cases. The data set obtained with SQ68 was analysed and split into pNA formed by free thrombin (⊗) and pNA formed by $\alpha_2$M-IIa (▽), using the algorithm described in Example 1.

It can be clearly seen that when using a substrate according to the present invention, experimental data are obtained in a very efficient way which represent directly the same biological activity (i.e. the amount of substrate split by free thrombin) as those which are obtained from a complex mathematical dissection of the curve from a conventional substrate.

## Claims

1. A process for the assessment of an active proteolytic enzyme in a blood or another biological fluid sample possibly comprising a complex of said proteolytic enzyme and $\alpha_2$-macroglobulin, **characterised in that** said sample is contacted with a substrate comprising a molecule having a $M_r$ larger than 10kD coupled to a signal-substrate, said signal-substrate comprising a detectable leaving group, wherein said substrate is hydrolysed by said proteolytic enzyme but not by said complex.

2. The process of claim 1, wherein the active proteolytic enzyme is selected from the group consisting of thrombin, activated clotting factor, activated fibrinolytic factor, and activated component of the complement system.

3. The process of claim 2, wherein the activated proteolytic enzyme is thrombin.

4. The process of any one of claims 1-3, wherein the molecule having a $M_r$ larger than 10kD is an inert molecule selected from the group consisting of protein, preferably ovalbumin, polysaccharide, and synthetic polymer.

5. The process of any one of the preceding claims, wherein the molecule having a $M_r$ larger than 10kD is water soluble.

6. The process of any one of claims 1-5, wherein the molecule having a $M_r$ larger than 10kD has a $M_r$ of about 40 kD and a solubility of at least 40 mg/ml.

7. The process of any one of claims 1-5, wherein the molecule having a $M_r$ larger than 10kD has a $M_r$ of about 20 kD and a solubility of at least 50 mg/ml.

8. The process of any one of the preceding claims, wherein the leaving group gives an optical density signal or a fluorescent signal.

9. The process of claim 8, wherein the leaving group is p-nitroanilide or 7-amino-4-methyl-coumarin.

10. The process of any one of the preceding claims, wherein the substrate is selected from the group consisting of ovalbumin coupled to H-D-isoteucyl-L-prolyl-L-arginine-p-nitroanilide.2HCl, ovalbumin coupled to H-alanyl-L-arginine-7-amido-4-methyl-coumarin, and ovalbumin coupled to H-D-phenylalanyl-L-prolyl-L-lysine-p-nitroanilide.2HCl.

11. The process of any one of claims 1-9, wherein the substrate comprises a leaving group of one or more hydrogen ions.

12. The process of any one of the preceding claims, wherein the amount and the kinetic properties of the substrate are selected such that the amount of proteolytic enzyme generated in the sample does not consume the substrate completely.

13. The process of any of the preceding claims, wherein the amount of hydrolysed substrate is directly proportional to the area under the generation curve of the proteolytic enzyme.

## Patentansprüche

1. Verfahren zur Bestimmung eines aktiven proteolytischen Enzyms in einer Blutprobe oder in einer anderen biologischen Flüssigkeitsprobe, die möglicherweise einen Komplex aus dem proteolytischen Enzym und $\alpha_2$-Makroglobulin umfasst, **dadurch gekennzeichnet, dass** die Probe mit einem Substrat in Kontakt gebracht wird, das ein Molekül mit einer $M_r$ von größer als 10kD, gekoppelt an ein Signalsubstrat, umfasst, wobei das Signalsubstrat eine nachweisbare Abgangsgruppe umfasst, und wobei das Substrat durch das proteolytische Enzym nicht aber durch den Komplex hydrolysiert wird.

**2.** Verfahren nach Anspruch 1, wobei das aktive proteolytische Enzym ausgewählt ist aus der Gruppe bestehend aus Thrombin, aktiviertem Gerinnungsfaktor, aktiviertem fibrinolytischen Faktor und einer aktivierten Komponente des Komplementsystems.

**3.** Verfahren nach Anspruch 2, wobei das aktivierte proteolytische Enzym Thrombin ist.

**4.** Verfahren nach einem der Ansprüche 1 bis 3, wobei das Molekül mit einer $M_r$ größer als 10kD ein inertes Molekül ist, ausgewählt aus der Gruppe bestehend aus Protein, vorzugsweise Ovalbumin, Polysaccharid und synthetischem Polymer.

**5.** Verfahren nach einem der vorstehenden Ansprüche, wobei das Molekül mit einer $M_r$ größer als 10kD wasserlöslich ist.

**6.** Verfahren nach einem der Ansprüche 1 bis 5, wobei das Molekül mit einer $M_r$ größer als 10kD eine $M_r$ von etwa 40kD und eine Löslichkeit von mindestens 40mg/ml aufweist.

**7.** Verfahren nach einem der Ansprüche 1 bis 5, wobei das Molekül mit einer $M_r$ größer als 10kD eine $M_r$ von etwa 20kD und eine Löslichkeit von mindestens 50mg/ml ausweist.

**8.** Verfahren nach einem der vorstehenden Ansprüche, wobei die Abgangsgruppe ein optische Dichte-Signal oder ein Fluoreszenzsignal ergibt.

**9.** Verfahren nach Anspruch 8, wobei die Abgangsgruppe p-Nitroanilid oder 7-Amino-4-methyl-cumarin ist.

**10.** Verfahren nach einem der vorstehenden Ansprüche, wobei das Substrat ausgewählt ist aus der Gruppe bestehend aus Ovalbumin gekoppelt an H-D-Isoleucyl-L-prolyl-L-arginin-p-nitroanilid·2HCl, Ovalbumin gekoppelt an H-Alanyl-L-arginin-7-amid-4-methyl-cumarin und Ovalbumin gekoppelt an H-D-Phenylalanyl-L-prolyl-L-lysin-p-nitroanilid·2HCl.

**11.** Verfahren nach einem der Ansprüche 1 bis 9, wobei das Substrat eine Abgangsgruppe von einem oder mehreren Wasserstoffionen umfasst.

**12.** Verfahren nach einem der vorstehenden Ansprüche, wobei die Menge und die kinetischen Eigenschaften des Substrats so ausgewählt sind, dass die Menge des proteolytischen Enzyms, das in der Probe erzeugt wird, das Substrat nicht vollständig aufbraucht.

**13.** Verfahren nach einem der vorstehenden Ansprüche, wobei die Menge des hydrolysierten Substrats direkt proportional zur Fläche unterhalb der Kurve der Erzeugung des protoelytischen Enzyms ist.

**Revendications**

**1.** Procédé de détermination d'une enzyme protéolytique active dans un échantillon de sang ou d'un autre liquide biologique susceptible de contenir un complexe de ladite enzyme protéolytique et d'$\alpha_2$-macroglobuline, **caractérisé en ce que** ledit échantillon est mis en contact avec un substrat comprenant une molécule ayant un $M_r$ de plus de 10 kD couplée à un substrat-signal, ledit substrat-signal comprenant un groupe partant détectable, dans lequel ledit substrat signal est hydrolysé par ladite enzyme protéolytique mais pas par ledit complexe.

**2.** Procédé selon la revendication 1, dans lequel l'enzyme protéolytique active est choisie dans le groupe constitué de la thrombine, le facteur de coagulation activé, le facteur fibrinolytique activé, et un composant activé du système complémentaire.

**3.** Procédé selon la revendication 2, dans lequel l'enzyme protéolytique activée est la thrombine.

**4.** Procédé selon l'une quelconque des revendications 1 à 3, dans lequel la molécule ayant un $M_r$ supérieur à 10 kD est une molécule inerte choisie dans le groupe constitué de protéines, de préférence de l'ovalbumine, des polysaccharides et des polymères synthétiques.

**5.** Procédé selon l'une quelconque des revendications précédentes, dans lequel la molécule ayant un $M_r$ supérieur à 10 kD est hydrosoluble.

**6.** Procédé selon l'une quelconque des revendications 1 à 5, dans lequel la molécule ayant un $M_r$ supérieur à 10 kD a un $M_r$ d'environ 40 kD et une solubilité d'au moins 40 mg/ml.

**7.** Procédé selon l'une quelconque des revendications 1 à 5, dans lequel la molécule ayant un $M_r$ supérieur à 10 kD a un $M_r$ d'environ 20 kD et une solubilité d'au moins 50 mg/ml.

**8.** Procédé selon l'une quelconque des revendications précédentes, dans lequel le groupe partant donne un signal de densité optique ou un signal fluorescent.

**9.** Procédé selon la revendication 8, dans lequel le groupe partant est la p-nitroanilide ou la 7-amino-4-méthylcoumarine.

**10.** Procédé selon l'une quelconque des revendications précédentes, dans lequel le substrat est choisi dans le groupe constitué de l'ovalbumine couplée à H-D-isoleucyl-L-prolyl-L-arginine-p-nitroanilide.2HCl, l'ovalbumine couplée à H-alanyl-L-arginine-7-amido-4-méthylcoumarine et l'ovalbumine couplée à H-D-phénylalanyl-L-prolyl-L-lysine-p-nitroanilide. 2HCl.

**11.** Procédé selon l'une quelconque des revendications 1 à 9, dans lequel le substrat comprend un groupe partant d'un ou plusieurs ions hydrogène.

**12.** Procédé selon l'une quelconque des revendications précédentes, dans lequel la quantité et les propriétés cinétiques du substrat sont choisies de telle sorte que la quantité d'enzyme protéolytique générée dans l'échantillon ne consomme pas complètement le substrat.

**13.** Procédé selon l'une quelconque des revendications précédentes, dans lequel la quantité de substrat hydrolysé est directement proportionnelle à la zone sous la courbe de génération de l'enzyme protéolytique.

**Figure 1**

**Figure 2**

**Figure 3**

**Figure 4**